# EUROPEAN PATENT APPLICATION

(11) **EP 4 230 166 A1**
(43) Date of publication of application: **23.08.2023**
(21) Application number: 22157361.1
(22) Date of filing: 17.02.2022
(51) Int. Cl.: A61B 18/20

(54) **LIGHT TREATMENT DEVICE**

(71) Applicant: Koninklijke Philips N.V., 5656 AG Eindhoven (NL)
(72) Inventor: BOAMFA, Marius Iosif, Eindhoven (NL); VAN ABEELEN, Frank Anton, Eindhoven (NL); VERHAGEN, Rieko, Eindhoven (NL); JUTTE, Petrus Theodorus, Eindhoven (NL)
(74) Representative: Philips Intellectual Property & Standards

(57) **Abstract**

A treatment device (2, 100) for performing a light-based treatment operation on or to a subject, the treatment device (2, 100) comprising: a light source (12, 101) configured to generate a light pulse, the light source (12, 101) comprising a gas; a plasma ignition unit (108) configured to apply a first voltage between the light source (12, 101) and a first electrode (102) to initiate plasma breakdown of the gas in the light source (12, 101); and a second electrode (106) connected to electrical ground; wherein the second electrode (106) is spaced from the first electrode (102) such that the first electrode (102) discharges via the second electrode (106) when the first voltage is applied.

## Description

### FIELD OF THE INVENTION

This disclosure relates to a treatment device for performing a light-based treatment operation on or to a subject.

### BACKGROUND OF THE INVENTION

Techniques for removal of unwanted hairs include shaving, electrolysis, plucking, laser and light therapies (known as photoepilation) and injection of therapeutic anti-androgens. Light-based technologies are also used in other types of dermatological treatments, including hair growth reduction and treating acne.

Light-based hair treatments inhibit the growth of hair by exposing the skin to bright flashes or pulses of light, known as intense pulsed light (IPL). Through the use of an appropriate configuration of the light energy, i.e. in terms of wavelength, intensity and/or pulse duration (if the light is to be pulsed), selective heating of the hair root and subsequent temporary or permanent damage to the hair follicle can be achieved. The IPL may be generated by a high intensity light source such as a gas discharge lamp (e.g., a Xenon flash lamp). The light penetrates the skin and is absorbed, among other places, in the root of the hair by the pigment melanin. This causes an increase in the temperature of the root of the hair to rise and subsequently the temperature of the surrounding tissue. The generated heat damages the hair follicles, and the growth of the hair is inhibited if the temperature rise is sufficient. This process is known as photothermolysis.

Light-based hair removal can be performed using commercially available 'home-use' devices (i.e. consumer devices that are suitable for use by non-specialists) such as the Philips Lumea device. Home-use devices typically use IPL technology at a relatively low fluence (e.g. up to 6.5J/cm²) compared to professional devices that use fluences in excess of 10J/cm². When the treatment is repeated in intervals of 2 to 4 weeks, a long-lasting hair reduction is achieved.

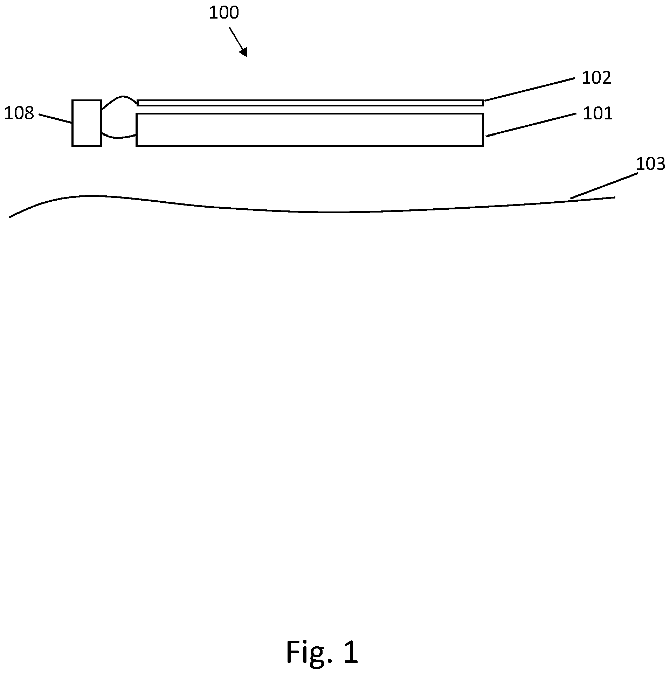

Fig. 1 is a schematic depicting some of the components of an IPL treatment device 100. The device 100 comprises a gas discharge lamp 101 for generating an intense light pulse (also referred to herein as a flash) and a light reflector 102 positioned to guide the light pulse towards the skin 103 of the subject. The gas discharge lamp 101 comprises a gas in a housing, e.g. a glass tube. The gas is typically a noble gas, such as Xenon or Argon, or a mixture of such gases. The flash is generated by applying a voltage across the gas to cause an electric discharge. The voltage may be applied, for example, via an electrode (not shown in Fig. 1) at either end of the glass tube. In most cases, an ignition voltage (known as a trigger) is first required to (partially) ionise the gas inside the lamp before the main electrical pulse initiates the main discharge that generates the flash. The partial ionisation of the gas is referred to as plasma breakdown. In some cases, the ignition voltage can be applied between an electrode in the glass tube and an additional electrode outside the glass tube (known as external triggering). The additional electrode (also referred to herein as the ignition electrode) can be any electrically conductive component, e.g. a metallic component. In the example shown in Fig. 1, the reflector 102, which is a metallic component, is used as the additional electrode such that the ignition voltage is applied between an electrode in the lamp 101 and the reflector 102. In another example, the additional electrode could be a metallic component that is wound around the glass tube. The metallic component may be in the form of a wire or strip. In Fig. 1, the ignition voltage between the lamp 101 and the reflector 102 is generated by a plasma ignition unit 108.

Treatment devices 100 may include various sensors that are not shown in Fig. 1. For example, the standard devices currently on the market typically include a skin contact sensor and a skin colour or skin tone sensor. These sensors are included, in part, for safety purposes. The skin colour/tone sensor is used to regulate the pulse energy according to the colour/tone of the area of the subject's skin, e.g., so that less energy is used for darker skin tones. The skin contact sensor is used to ensure that the flash is enabled only when the device is correctly in contact with the treatment area. These and other sensors may be incorporated into the treatment device or provided as attachments for the device.

Imaging sensors (e.g. in a camera) are also increasingly being included in treatment devices 100 to obtain images of the area that is to be treated and/or that has been treated. Indeed, future standards and regulations appear to be evolving towards the inclusion of an imaging sensor in the IPL optical engine. The imaging sensor may be used to image the treatment area before the IPL flash is deployed to determine whether it is safe to perform the treatment on the imaged area. This could include detecting the skin tone of the area, the condition of the skin, and the presence of any tattoos, moles and/or other skin features that could impact on the suitability and/or safety of the light-based treatment. Other potential uses of an imaging sensor embedded in the treatment device could include treatment guidance via displacement measurement and evaluating the effects of a treatment.

### SUMMARY OF THE INVENTION

The high ignition voltage used to initiate the plasma breakdown in IPL treatment devices can put sensors and/or metallic components that are incorporated into the device near to the gas discharge lamp at risk. The size of the voltage that is required to ionise the gas in a gas discharge lamp is determined by the type of gas and the pressure of the gas. Typical ignition voltages for IPL devices are in region of 12 to 17 kV. At these voltages, nearby sensors/components are prone to capacitive coupling with the ignition electrode (i.e. the additional electrode outside the glass tube) as well as being at risk of a direct voltage discharge from the ignition electrode to the sensor/component.

This problem has particularly been observed with the cameras that are increasingly being incorporated into treatment devices. An imaging sensor in an IPL device is preferably positioned close to the gas discharge lamp so that images of the area to be/being treated can be obtained. However, placing the imaging sensor close to the gas discharge lamp leads to capacitive coupling between the imaging sensor and the ignition electrode which can prevent the normal functioning of the imaging sensor. A direct discharge from the high voltage ignition electrode to the imaging sensor will also damage the imaging sensor and could lead to camera failure.

Therefore, it is desirable to provide a treatment device that reduces the risk of electrical damage within the device due to the high voltage used to initiate plasma breakdown in the gas discharge lamp. Such a treatment device will be more suitable for the incorporation of additional sensors and components (e.g., cameras) than the treatment devices that are currently available. Thus, it is an object of the present disclosure to provide a light-based treatment device in which an imaging sensor, as well as other types of sensors or electrical components, can be incorporated close to the gas discharge lamp without compromising the functionality of the device and/or the sensor(s)/component(s).

According to the disclosure herein, it is proposed to position an earthed electrode within the treatment device and close enough to (but not in contact with) the ignition electrode to provide a path to electrical ground. Thus, each time the ignition voltage is applied to enable a light pulse to be generated, a controlled, high voltage discharge takes place through the earthed electrode. It has been found that this controlled discharge does not prevent the light source from emitting the light pulse.

According to a first aspect, there is provided a treatment device for performing a light-based treatment operation on or to a subject. The treatment device comprises a light source configured to generate a light pulse, the light source comprising a gas; a plasma ignition unit configured to apply a first voltage between the light source and a first electrode to initiate plasma breakdown of the gas in the light source; a light exit window through which the light pulse is emitted from the treatment device; and a second electrode connected to electrical ground. The second electrode is spaced from the first electrode such that the first electrode discharges via the second electrode when the first voltage is applied.

Thus, the present invention provides a treatment device in which the risk of electrical damage to sensors and/or other electrical components that are within the device is reduced. This is achieved without compromising the functionality of the treatment device and/or the sensor(s) therein. The invention can enable sensors such as imaging sensors to be positioned close to the light source so that the imaging sensor can obtain images of the area being treated with the light pulse. Alternatively or in addition, the invention can provide greater freedom in designing treatment devices, since sensors and/or electrical components can be positioned closer to the light source than in conventional treatment devices. The earthed electrode provides for a controlled (and predictable) discharge during every light pulse and prevents an uncontrolled discharge that could damage nearby sensors/components. The present disclosure also reduces the length of time that capacitive coupling between the ignition electrode and sensor(s) in the device takes place.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic showing some of the components of a treatment device;
Fig. 2 is an illustration of an exemplary treatment device;
Fig. 3 is a schematic demonstrating the problem that can arise in treatment devices;
Fig. 4 is a schematic of parts of a treatment device according to various embodiments;
Fig. 5 is a schematic of parts of a treatment device according to various embodiments; and
Fig. 6 is a schematic of parts of a treatment device according to various embodiments.

### DETAILED DESCRIPTION OF THE EMBODIMENTS

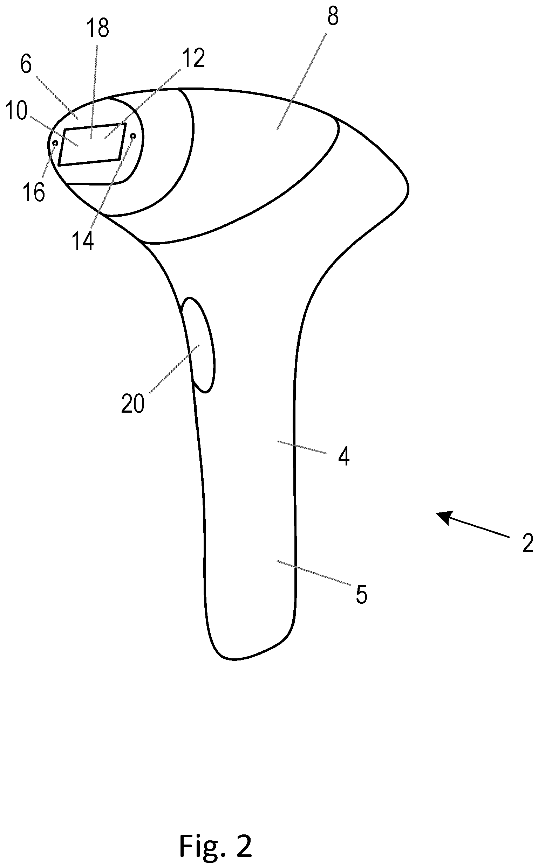

Fig. 2 is an illustration of an exemplary treatment device 2 that can be used to apply a light pulse to an area of skin. It will be appreciated that the treatment device 2 in Fig. 2 is merely presented as an example of a hand-held treatment device 2 that the invention can be used with, and the treatment device 2 is not limited to the form shown in Fig. 2 or to being a hand-held treatment device. The treatment device 2 is for use on a body of a subject (e.g. a person or an animal), and is to be held in one or both hands of a user during use. The treatment device 2 is to perform some treatment operation to hairs on the body of the subject using one or more light pulses when the treatment device 2 is in contact with a body part of the subject. The treatment operation can be removal of unwanted hairs by laser and/or light therapies (known as a photoepilation treatment or Intense Pulsed Light treatment).

As described herein, the treatment device 2 is operated or used by a 'user', and the treatment device 2 is used on a body of a 'subject'. In some cases, the user and the subject is the same person, i.e. the treatment device 2 is held in a hand and used by a user on themselves (e.g. used on the skin on their leg). In other cases, the user and the subject are different people, e.g. the treatment device 2 is held in a hand and used by a user on someone else.

The exemplary treatment device 2 comprises a housing 4 that includes at least a handle portion 5 and a head portion 6. The handle portion 5 is shaped to enable the user to hold the treatment device 2 with one hand. The head portion 6 is at a head end 8 of the housing 4, and the head portion 6 is to be placed into contact with the subject in order for the personal care operation to be performed on the body or skin of the subject at the position that the head portion 6 is in contact with the body or skin.

The treatment device 2 is for performing a treatment operation using light pulses. Thus, in Fig. 2 the head portion 6 comprises an aperture 10, also referred to as a light exit window, that is arranged in or on the housing 4 so that the aperture 10 can be placed adjacent to or on (i.e. in contact with) the skin of the subject. The treatment device 2 includes one or more light sources 12 that are for generating light pulses that are to be applied to the skin of the subject via the aperture 10 and effect a treatment operation. The one or more light sources 12 are arranged in the housing 4 so that the light pulses are provided from the one or more light sources 12 through the aperture 10. The aperture/light exit window 10 may be in the form of an opening at the head end 8 of the housing 4, or it may be in the form of a window (including a waveguide) that is transparent or semi-transparent to the light pulses (i.e. the light pulses can pass through the window).

In the exemplary embodiment shown in Fig. 2, the aperture 10 has a generally rectangular shape, which results in a generally rectangular-shaped skin treatment region on the skin. It will be appreciated that the aperture 10 can have any other desired shape. For example the aperture 10 can be square, elliptical, circular, or any other polygonal shape.

The one or more light sources 12 can generate light pulses of any suitable or desired wavelength (or range of wavelengths) and/or intensities. For example, the light source 12 can generate visible light, infra-red (IR) light and/or ultraviolet (UV) light. Each light source 12 can comprise any type of light source for which a high voltage is to be applied in order to enable a light pulse to be generated, such as a gas discharge lamp.

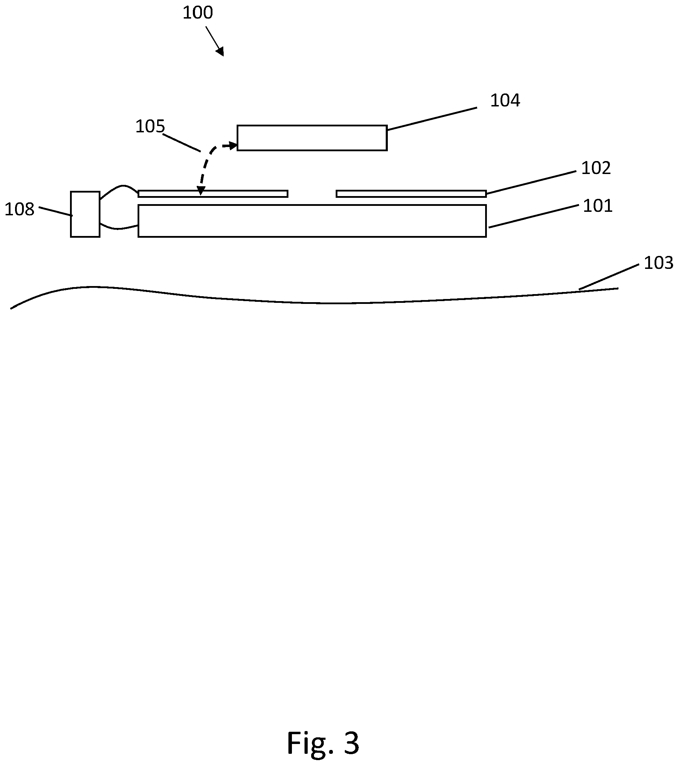

According to the embodiments herein, at least one of the one or more light sources is a gas discharge lamp (e.g. a Xenon flash lamp). The gas discharge lamp may comprise a gas in a housing (e.g. a glass tube), where the gas is typically a noble gas, such as Xenon or Argon, or a mixture of such gases. In these embodiments, the treatment device 2 also includes a plasma ignition unit for providing a high voltage to initiate plasma breakdown of the gas in the light source. This process is further described with reference to Fig. 3.

In some embodiments, the light source(s) 12 generate UV, visible and/or IR light, and a filter may be used to absorb some wavelengths. For example, the filter may remove UV light and visible light at the blue end of the spectrum. In some embodiments, light with wavelengths of less than 560 nm may be filtered out. The resulting light pulse(s) may be emitted from the device with spectral content in the 560-1800 nanometre (nm) range. These wavelengths (in particular wavelengths in the range 560 to 1200 nm) heat melanin in the hair and hair root by absorption, which puts the hair follicles in a resting phase, preventing hair regrowth.

The one or more light sources 12 are configured to provide pulses of light. That is, the light source(s) 12 are configured to generate light at a high intensity for a short duration (e.g. less than 1 second). In some examples, the light pulse may have a duration between 4 and 8 milliseconds (ms). The Full Width Half Maximum (FWHM) of the light pulse may be about 1.8 ms. The intensity of the light pulse should be high enough to effect the treatment operation on the skin or body part adjacent the aperture 10. For example, home-use devices can provide light pulses at fluences of up to 6.5 J/cm², while professional devices can provide light pulses at fluences in excess of 10 J/cm².

The illustrated treatment device 2 also includes two skin contact sensors 14, 16 positioned on or in the head portion 6 that are used to determine whether the head portion 6 is in contact with the skin before a light pulse is generated to avoid the light pulse being directed into the eyes of the user or subject.

The illustrated treatment device 2 includes an imaging sensor 18 that is located in the head portion 6 and is positioned close to one or more of the light source(s) 12. In some embodiments, the imaging sensor 18 can be configured to obtain images of the area of skin that: is to be treated, is being treated or has been treated. For example, the imaging sensor 18 may be used to determine the condition of the skin to be treated and/or whether the treatment area includes any tattoos, moles and/or other skin features that could impact on the suitability and/or safety of the light-based treatment.

Alternatively, or in addition, the imaging sensor 18 may be configured to determine a skin tone of the skin that is to be treated. The skin tone may be determined to make sure that the light pulse has an intensity that is appropriate for the type of skin being treated, or even to prevent a light pulse being generated if the skin type is unsuitable for light pulses (e.g. darker skin which has a much higher melanin content). The imaging sensor 18 may determine the skin tone by measuring an intensity or level of light at a particular wavelength or multiple wavelengths reflected from the skin. The measured intensity or level of reflected light at a particular wavelength(s) can be indicative of the skin tone. The measured intensity or level of reflected light can be based on the concentration of melanin in the skin, and thus the measured intensity or level can indicate the melanin concentration. The melanin concentration can be derived, for example, from measurements of light reflection at 660nm (red) and 880nm (infrared) wavelengths.

In some embodiments, the imaging sensor 18 may be configured to determine whether the head portion 6 of the device is in contact with the skin of a subject. In these embodiments, the imaging sensor 18 may be used instead of, or as well as, the skin contact sensors 14, 16 described above.

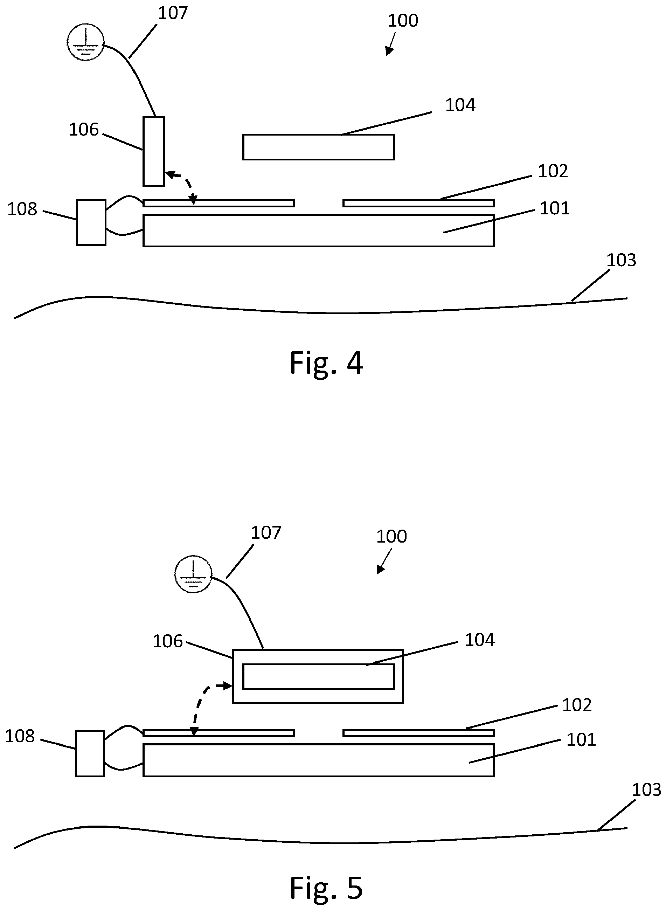

The imaging sensor 18 may be, for example, the imaging sensor 104 described with reference to any of Figs. 4-6.

The illustrated treatment device 2 also includes a user control 20 that can be operated by the user to activate the treatment device 2 so that the head portion 6 performs the required treatment operation on the body of the subject (e.g. the generation of one or more light pulses by the one or more light source(s) 12). The user control 20 may be in the form of a switch, a button, a touch pad, etc.

As noted above, there are challenges associated with incorporating an imaging sensor and/or other sensors in IPL treatment devices such as those depicted in Figs. 1 and 2. These challenges are described with reference to Fig. 3.

The part of the treatment device 100 that was depicted in Fig. 1 is shown in Fig. 3 with an imaging sensor 104 positioned close to the lamp 101 and the reflector 102. There is an opening in the reflector 102 so that the imaging sensor 104 can obtain images of the skin 103 of a subject via a light exit window (not shown) and the (e.g. glass) housing of the lamp 101. In this example, the lamp 101 is a gas discharge lamp, such as a Xenon flash lamp.

As described with reference to Fig. 1, the device 100 comprises a plasma ignition unit 108 that is configured to, at the beginning of every flash, apply an ignition voltage between the reflector 102 and an electrode of the lamp 101 (that form an open circuit). The electrodes of the lamp 101 are not shown in Fig. 3. The ignition voltage must be sufficiently high to form a plasma, i.e., to ionise the gas atoms and/or molecules in the gas discharge lamp 101. For typical IPL devices, the ignition voltage is in the range 5 to 30 kV. Preferably, the ignition voltage is in the range 12 to 17 kV. In some examples, the ignition voltage is 15 kV.

When the reflector 102 is placed under high voltage (e.g. when the reflector 102 is acting as an electrode for the ignition voltage), capacitive coupling occurs between the reflector 102 and any electrically conductive components in its vicinity. Therefore, the configuration shown in Fig. 3 leads to capacitive coupling 105 between the reflector 102 and the imaging sensor 104. The imaging sensor 104 is also at risk of receiving a direct discharge from the reflector 102. Such capacitive coupling and/or voltage discharge (depicted in Fig. 3 by the dashed arrow 105) can prevent the normal functioning of the imaging sensor and can lead to short- or long-term damage to the imaging sensor comprised therein.

To address these and other problems, there is provided a treatment device for performing a light-based treatment operation on or to a subject. The treatment device comprises: a light source comprising a gas; a plasma ignition unit configured to apply a first voltage between the light source and a first electrode to initiate plasma breakdown of the gas in the light source; and a second electrode connected to electrical ground. The second electrode is spaced from (i.e. not in contact with) the first electrode such that the first electrode discharges via the second electrode when the first voltage is applied.

The treatment device may be an Intense Pulsed Light, IPL, device for performing IPL photo-epilation. The light source may be a gas discharge lamp such as a flash lamp. Examples of gases used in flash lamps include a Xenon, Argon and Krypton. In some embodiments, the flash lamp may comprise a molecular gas and/or a mixture of gases.

The light source may comprise a housing (e.g. a glass tube) in which the gas is contained. The light source may further comprise at least two electrodes (distinct from the first and second electrode already mentioned) that are spaced from each other such that there is gas between them.

The plasma ignition unit is configured to apply a first voltage (also referred to herein as an ignition voltage) between the light source and the first electrode (also referred to herein as an ignition electrode) to initiate plasma breakdown of the gas in the light source. The first voltage may be between one of the electrodes comprised in the light source and the first electrode. In some embodiments, the first voltage is between 5 and 30 kV. Preferably, the first voltage is between 12 and 17 kV.

The first electrode can be any suitable electrically conductive component. For example, in some embodiments the first electrode is part of a reflector that is also used for guiding the light pulse to the light exit window. In other embodiments, the first electrode is part of a metal component (e.g. a metal wire) that is wound around the light source.

The first electrode is comprised in the treatment device. In some embodiments, the first electrode is positioned outside the light source for external triggering of the light source. In these embodiments, the first electrode may be in contact with the light source (e.g. in contact with the housing (e.g. glass tube)), or spaced from, but proximal to (i.e. in the close vicinity of) the light source. In some embodiments, 'proximal to' could mean spaced from the light source by between 0.1 and 2 mm.

Although not shown in Fig. 3, the treatment device comprises a light exit window through which the light pulse is emitted from the treatment device. The light exit window may be an aperture, e.g. a hole/opening in the housing of the treatment device. Alternatively, the light exit window may be a translucent or transparent material (e.g. glass or plastic) through which light can travel.

In some embodiments, the treatment device further comprises a sensor. For example, the sensor could be an imaging sensor for obtaining one or more images of an area or region near to the treatment device, for example an area of skin that is to be treated using the light pulse or an area of skin that has been treated using the light pulse. The imaging sensor obtains one or more images using light passing into the treatment device. Alternative types of sensors include a skin contact sensor or a skin tone sensor. The sensor could be for sensing one or more of: skin contact, skin colour, hair colour, moles, tattoos, and skin condition. In the case of an imaging sensor, the image(s) obtained by the imaging sensor can be analysed to determine any one or more of skin contact, skin colour, hair colour, moles, tattoos, and skin condition.

In embodiments where a sensor is present in the treatment device, at least a part of the second electrode is closer to the first electrode than any part of the sensor. In other words, at least a part of the second electrode is spaced from the first electrode by a smaller distance than the smallest distance between the sensor and the first electrode. This ensures that the first voltage discharges to ground via the second electrode and not the sensor.

In some embodiments, the smallest distance between the second electrode and the first electrode is equal to or smaller than an air breakdown distance for the first voltage at atmospheric pressure. This positioning reduces the risk of electrical damage to the sensor. Each time the first (ignition) voltage is applied, a high voltage discharge takes place to the earthed second electrode. This reduces the length of time of capacitive coupling into the sensor. Furthermore, the high voltage discharge to the earthed electrode prevents a high voltage discharge to the sensor.

The inclusion of the second electrode provides for a controlled voltage discharge each time a flash in generated. The ignition voltage (e.g. approximately 15 kV) is required to initiate plasma breakdown in the flash lamp. As such, it may be expected that a controlled discharge of this voltage would compromise the normal functioning of the flash lamp. In other words, it may be expected that if the ignition voltage discharges, then the ignition voltage will not be present to (partially) ionise the gas in the light source (i.e. to initiate plasma breakdown), and therefore the flash will not be generated. However, experimental observation shows that the controlled discharge does not prevent the emission of the intense light pulse, provided the earthed (second) electrode is not in conductive contact with the ignition (first) electrode (i.e. provided there is not a conductive path between the ignition electrode and the earthed electrode). The experimental observations indicate that if the ignition electrode is spaced from the earthed electrode, the time needed to initiate plasma breakdown is less than the time needed for the controlled discharge of the ignition voltage. Therefore, the controlled discharge does not prevent plasma breakdown. Conversely, if the ignition electrode is in conductive contact with the earthed electrode, then the ignition voltage discharges directly to ground, and no plasma breakdown can occur, thereby preventing the generation of the IPL flash.

As disclosed herein, the inclusion of an earthed electrode that is spaced from the first electrode provides a path for current to flow to ground in a controlled manner. This controlled discharge prevents the imaging sensor or any other components in the device from becoming a high voltage discharge path and thus protects such components from electrical damage. The earthed electrode also reduces the length of time of any capacitive coupling between: (i) the ignition electrode in the lamp, and (ii) the imaging sensor or other sensor(s)/component(s). As such, sensors (such as an imaging sensor) and/or other components can be included in the treatment device with a reduced risk of electrical damage to the sensors/components, and without compromising the functionality of the treatment device.

Specific embodiments of the invention are depicted schematically in Figs. 4, 5 and 6.

Fig. 4 shows a treatment device 100 with an imaging sensor 104 positioned close to the lamp 101 and the reflector 102. The imaging sensor is configured to obtain one or more images using light passing into the treatment device. The obtained images could be, for example, images of the skin 103 before it is to be treated and/or after it has been treated. It will be understood that the invention is equally applicable to other sensors or components in place of, or in addition to, the imaging sensor 104.

The reflector 102 in Fig. 4 has a dual function. Firstly, it reflects the treatment light pulse towards the skin 103 of a subject. Secondly, it acts as a high voltage electrode for the ignition voltage that is used to initiate plasma breakdown in the lamp 101. The treatment device 100 also comprises a second electrode 106 that is connected to electrical earth 107. The second electrode 106 is spaced from the reflector 102 such that, each time the plasma ignition unit 108 applies the first voltage (i.e. the ignition voltage) between the reflector 102 and the lamp 101, the high voltage in the reflector 102 discharges via the second electrode 106. It has been experimentally observed that this high voltage discharge to an earthed electrode 106 does not prevent the proper functioning of the lamp 101 and, thus, does not prevent the IPL emission.

The smallest distance between the second electrode 106 and the reflector 102 is equal to or smaller than an air breakdown distance for the first voltage at atmospheric pressure. This ensures that every time the ignition voltage is applied, a high voltage discharge to the second electrode 106 takes place.

Fig. 5 shows an alternative embodiment in which the second electrode 106 is a case or housing in which the imaging sensor 104 is disposed, e.g., a metallic case around a camera. In other embodiments, the second electrode 106 could be a case in which any other type of sensor is disposed. The case 106 is connected to electrical earth 107, electrically isolated from the imaging sensor 104 contained therein, and is spaced from the reflector 102 such that, each time the first voltage (i.e. the ignition voltage) is applied between the reflector 102 and the lamp 101, the reflector 102 discharges via the second electrode 106. The current flows to ground in a controlled manner and therefore does not pass through the imaging sensor 104 (or other sensor(s)).

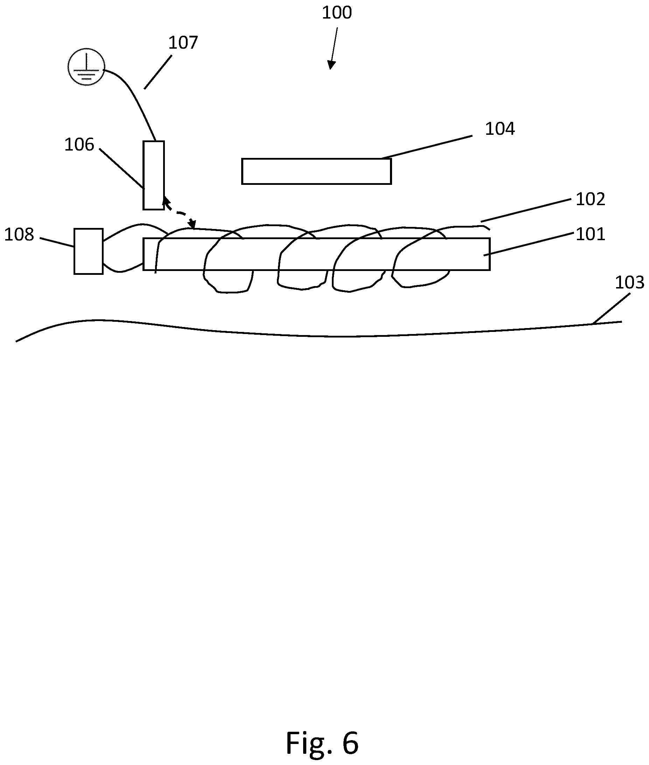

Fig. 6 shows an alternative embodiment in which the first electrode is a metal wire 102 wound around the lamp 101. In this way, the metal wire 102 (or similar type of metallic component, such as a strip) can act as an electrode for the ignition voltage. Each time the plasma ignition unit 108 applies the ignition voltage between the lamp 101 and the metal wire 102, a controlled discharge takes place to the earthed second electrode 106, thereby protecting the imaging sensor 104.

Therefore, there is provided a treatment device for performing a light-based treatment operation on or to a subject, where the treatment device is configured so that the risk of electrical damage to sensors and/or components in the vicinity of the light source comprised therein is reduced compared to conventional treatment devices.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A treatment device (2, 100) for performing a light-based treatment operation on or to a subject, the treatment device (2, 100) comprising:
a light source (12, 101) configured to generate a light pulse, the light source (12, 101) comprising a gas;
a plasma ignition unit (108) configured to apply a first voltage between the light source (12, 101) and a first electrode (102) to initiate plasma breakdown of the gas in the light source (12, 101); and
a second electrode (106) connected to electrical ground;
wherein the second electrode (106) is spaced from the first electrode (102) such that the first electrode (102) discharges via the second electrode (106) when the first voltage is applied.

2. A treatment device (2, 100) as defined in claim 1, wherein the treatment device (2, 100) further comprises a sensor.

3. A treatment device (2, 100) as defined in claim 2, wherein the sensor is an imaging sensor for obtaining one or more images using light passing into the treatment device (2, 100).

4. A treatment device (2, 100) as defined in claim 2 or 3, wherein the sensor is for sensing one or more of: skin contact, skin colour, hair colour, moles, tattoos, and skin condition.

5. A treatment device (2, 100) as defined in any of claims 2-4, wherein the second electrode (106) is a case in which the sensor is disposed.

6. A treatment device (2, 100) as defined in any of claims 2-5, wherein at least a part of the second electrode (106) is closer to the first electrode (102) than any part of the sensor.

7. A treatment device (2, 100) as defined in any of claims 2-6, wherein the sensor is arranged within the treatment device (2, 100) to sense one or more parameters of the subject via a light exit window (10) through which the light pulse is emitted from the treatment device (2, 100).

8. A treatment device (2, 100) as defined in any of claims 1-7, wherein the first electrode (102) is a metallic component.

9. A treatment device (2, 100) as defined in any of claims 1-8, wherein the first electrode (102) is a reflector for guiding the light pulse to the light exit window (10).

10. A treatment device (2, 100) as defined in any of claims 1-9, wherein the smallest distance between the second electrode (106) and the first electrode (102) is equal to or smaller than an air breakdown distance for the first voltage at atmospheric pressure.

11. A treatment device (2, 100) as defined in any of claims 1-10, wherein the first voltage is between 12 and 17 kV.

12. A treatment device (2, 100) as defined in any of claims 1-11, wherein the light source (12, 101) is a gas discharge lamp.

13. A treatment device (2, 100) as defined in claim 12, wherein the gas discharge lamp is a flash lamp.

14. A treatment device (2, 100) as defined in any of claims 1-13, wherein the treatment operation is Intense Pulsed Light, IPL, photo-epilation.
